# EUROPÄISCHE PATENTSCHRIFT

(11) **EP 3 907 142 B1**
(45) Veröffentlichungstag und Bekanntmachung des Hinweises auf die Patenterteilung: **30.04.2025**
(21) Anmeldenummer: 20173245.0
(22) Anmeldetag: 06.05.2020
(51) Int. Cl.: B65B 3/00, A61M 39/12, B65B 3/04, B65B 39/00, F16L 33/00, A61J 1/20

(54) **VORRICHTUNG ZUM BEFÜLLEN VON INFUSIONSBEUTELN**
DEVICE FOR FILLING INFUSION BAGS
DISPOSITIF DE REMPLISSAGE DE POCHES DE PERFUSION

(43) Veröffentlichungstag der Anmeldung: 10.11.2021
(73) Patentinhaber: Harro Höfliger Verpackungsmaschinen GmbH, 71573 Allmersbach im Tal (DE)
(72) Erfinder: Wolf, Stefan, 71573 Allmersbach im Tal (DE); Stahl, Ulrich, 71573 Allmersbach im Tal (DE)
(74) Vertreter: Schmid, Barbara

(56) Entgegenhaltungen:
- EP-A1- 1 411 023
- EP-A1- 1 860 057
- WO-A1-2012/061359
- DE-B3- 102015 106 125
- US-A- 3 299 603

## Beschreibung

### TECHNISCHES GEBIET

Die Erfindung betrifft eine Vorrichtung zum Befüllen von Infusionsbeuteln, insbesondere zum Befüllen von Infusionsbeuteln mit einer Flüssigkeit. Bei den Infusionsbeuteln kann es sich um Beutel mit lediglich einer Kammer handeln, aber auch um Mehrkammerbeutel. Mehrkammerbeutel können beispielsweise auch ein Pulver und eine Liquidkammer umfassen.

### STAND DER TECHNIK

Die Befüllung von Infusionsbeuteln muss in der Regel unter besonderen Bedingungen ("low bio burden" genannt) erfolgen, da der in den Infusionsbeuteln enthaltene Inhalt einem Patienten zugeführt wird und daher nicht kontaminiert werden darf. Bei kritischen Inhaltsstoffen, insbesondere bei Krebsmedikamenten oder auch bei Infusionen zur parenteralen Ernährung, kann eine Abfüllung der Infusionsbeutel unter Inertgas notwendig sein, da die abzufüllende Flüssigkeit nicht mit Luft-Sauerstoff in Kontakt kommen darf. Dabei wird in der Regel Stickstoff als Inertgas verwendet.

Eine Abfüllung von Flüssigkeiten unter Inertgas-Bedingungen kann beispielsweise durch eine Dachbegasung erfolgen. In diesem Fall wird die Vorrichtung zum Befüllen der Infusionsbeutel in einem konstanten Strom aus Inertgas platziert. Bei dieser Methode werden jedoch große Mengen an Stickstoff verbraucht, so dass erhebliche Kosten entstehen. Darüber hinaus kommt es zu einer sicherheitstechnischen Problematik, da die großen Mengen an Stickstoff häufig bauartbedingt unkontrolliert abfließen. Darüber hinaus wird der kritische Beutelbereich abgeschattet, so dass gegebenenfalls kein optimaler laminarer Luftstrom im kritischen Bereich der Füllschläuche erreicht werden kann.

Alternativ zu der Dachbegasung kann für die Befüllung von Infusionsbeuteln unter Inertgas-Bedingungen ein spezieller Füllkopf verwendet werden, bei dem mehrere Medien durch die Füllnadel geleitet werden können. Auf diese Weise kann eine Nachbegasung mit Stickstoff möglich sein. Auch eine Evakuierung der Füllschläuche vor dem eigentlichen Befüllen wäre auf diese Weise möglich. Bei einem solchen Füllkopf für mehrere Medien gestaltet sich die Reinigung des Füllkopfes jedoch sehr schwierig. Insbesondere durch die Möglichkeit des Einzugs von Rest-Flüssigkeit in den Vakuumkreis ist eine intensive Reinigung des Füllkopfes jedoch unerlässlich. Die Nutzung der Füllnadel für Vakuum und/ oder Stickstoffbegasung neben der eigentlichen Verwendung zur Befüllung der Infusionsbeutel mit einer Flüssigkeit erfordert die vollständige Entleerung der Füllnadel vor dem Wechsel der Anwendung. Unter Berücksichtigung der vorgesehenen Füllmedien ist dies unerwünscht und technisch sehr aufwendig. Die Restmengen der Füllmedien müssen separat gesammelt und entsorgt werden, da keine Entsorgung über das Abwasser möglich ist.

Aus der US 3,299,603 ist eine Vorrichtung zur Befüllung von Infusionsbeuteln mit einer Flüssigkeit bekannt. Die Vorrichtung besitzt eine Füllnadel und einen Grundhalter. Der Grundhalter verläuft um die Füllnadel herum, wobei zwischen dem Grundhalter und der Füllnadel ein umlaufender Zwischenraum vorhanden ist. Darüber hinaus ist ein Gasanschluss vorhanden, durch den Inertgas durch die Füllnadel geleitet werden kann. Eine ähnliche Vorrichtung ist auch aus der WO 2012/ 061359 A1 bekannt.

Aus der EP 1 411 023 A1 und der DE 10 2015 106 125 B3 sind Vorrichtungen zur Abfüllung von Getränken bekannt, die ebenfalls eine Füllnadel und einen Grundhalter besitzen.

### DARSTELLUNG DER ERFINDUNG

Ausgehend von diesem vorbekannten Stand der Technik liegt der Erfindung die Aufgabe zugrunde, eine verbesserte Vorrichtung zum Befüllen von Infusionsbeuteln anzugeben, bei der eine Befüllung unter Inertgas-Bedingungen prozesssicher möglich ist.

Die erfindungsgemäße Vorrichtung zum Befüllen von Infusionsbeuteln ist durch die Merkmale des Hauptanspruchs 1 gegeben. Sinnvolle Weiterbildungen der Erfindung sind Gegenstand von sich an diesen Anspruch anschließenden weiteren Ansprüchen.

Die erfindungsgemäße Vorrichtung zum Befüllen eines Infusionsbeutels besitzt eine Füllnadel und einen Grundhalter, der so um die Füllnadel herum verläuft, dass zwischen der Füllnadel und dem Grundhalter ein umlaufender Zwischenraum vorhanden ist. Darüber hinaus ist zumindest ein Gasanschluss für Inertgas vorhanden, durch den Inertgas in den umlaufenden Zwischenraum zwischen der Füllnadel und dem Grundhalter geleitet werden kann. Am unteren Ende des Grundhalters ist eine bewegliche Düse vorhanden, die längsverschieblich gelagert ist und in ihrer Ruhestellung über das untere Ende der Füllnadel hinausragt.

Die Füllnadel weist erfindungsgemäß einen radial auskragenden Anschlussflansch auf. Über diesen Anschlussflansch kann die Füllnadel an einer Befüllvorrichtung angeschlossen werden. Auch der Grundhalter weist einen radial auskragenden Anschlussflansch auf, so dass eine Befestigung des Grundhalters an der Füllnadel über die jeweiligen Anschlussflansche möglich ist.

Die Füllnadel selbst wird erfindungsgemäß somit lediglich für die Befüllung der Infusionsbeutel mit der zu befüllenden Flüssigkeit verwendet. Die verwendeten Medien (Inertgas beziehungsweise zu befüllende Flüssigkeit) werden daher durch unterschiedliche Kanäle geleitet, so dass keine Vermischung der Medien stattfindet. Dies führt zu einer vergleichsweise einfachen Maschinenkonstruktion. Die erfindungsgemäße Ausgestaltung der Vorrichtung zum Befüllen von Infusionsbeuteln ermöglicht daher eine effektive und prozesssichere Reinigung der Vorrichtung, insbesondere der Füllnadel.

Durch die Verwendung der erfindungsgemäßen Vorrichtung können Einkammerbeutel und Mehrkammerbeutel mit einem deutlich reduzierten Rest-Sauerstoffgehalt im Headspace hergestellt werden. Dabei können deutliche reduzierte Zykluszeiten realisiert werden, so dass ein sicherer und wirtschaftlicher Betrieb der erfindungsgemäßen Vorrichtung möglich ist.

Die voreilende bewegliche Düse kann insbesondere federbelastet sein und mittels einer Druckfeder längsverschieblich gelagert sein. Eine solche Ausführungsform ist einfach und wirtschaftlich umsetzbar und kann im Bedarfsfall unkompliziert ausgetauscht werden.

Die Form der Füllnadel selbst, insbesondere des unteren Endes der Füllnadel, die in den Infusionsbeutel eingeführt wird, wird durch die erfindungsgemäße Vorrichtung nicht eingeschränkt. Insofern sind nach wie vor unterschiedliche äußere Geometrien der Füllnadel möglich, so dass beispielsweise eine Anpassung der Füllnadel an die Form des Anschlusses der Infusionsbeutel möglich ist.

In der Regel wird der zu befüllende Infusionsbeutel an einer vorgelagerten Station mit Inertgas, insbesondere mit Stickstoff gespült werden. Der gespülte Infusionsbeutel wird anschließend leergedrückt und der Füllschlauch des Infusionsbeutels abgeklemmt. Oberhalb der Abklemmung bleibt ein Stück Füllschlauch stehen. Dieses Stück Füllschlauch kann in Kontakt mit der Umgebungsluft kommen und dadurch für unzulässig hohe Sauerstoffwerte bei der Abfüllung der Infusionsbeutel sorgen.

Bei der Befüllung eines Infusionsbeutels kann zunächst lediglich das Inertgas durch den umlaufenden Zwischenraum und die bewegliche Düse geleitet werden. Auf diese Weise kann Inertgas in den oberhalb der Abklemmung verbleibenden Füllschlauch des Infusionsbeutels geleitet werden, um die Umgebungsluft aus dem Füllschlauch zu verdrängen. Anschließend kann die bewegliche Düse ein Stück weit nach oben zurückgeschoben werden. Vorzugsweise kann die bewegliche Düse dabei so weit nach oben zurückgeschoben werden, dass diese oberhalb des unteren Endes der Füllnadel vorhanden ist. Dadurch kann die Füllnadel zur Befüllung des Infusionsbeutels mit der zu befüllenden Flüssigkeit in direkten Kontakt mit dem Füllschlauch des Infusionsbeutels gebracht werden, so dass kein Kontakt der zu befüllenden Flüssigkeit mit Umgebungsluft möglich sein sollte.

Um die bewegliche Düse aus ihrer Ruhestellung nach oben zu verschieben, kann in einer ersten Ausführungsform ein Anschlagelement vorhanden sein. Sofern die Vorrichtung höhenverstellbar ausgebildet ist, kann bei einem Absenken der Vorrichtung zunächst ein Kontakt mit dem Anschlagelement erfolgen, durch das die bewegliche Düse beim weiteren Absenken der Vorrichtung entsprechend nach oben verschoben werden kann. In einer zweiten Ausführungsform kann die bewegliche Düse durch einen motorischen oder pneumatischen Antrieb aus ihrer Ruhestellung nach oben verschoben werden. Eine solche Ausbildung wäre unabhängig von einer möglichen Höhenverstellbarkeit der Vorrichtung.

Grundsätzlich kann ein einzelner Gasanschluss für Inertgas ausreichend sein, um den umlaufenden Zwischenraum mit Inertgas zu befüllen und ausreichend Inertgas über die bewegliche Düse auszuströmen. Vorzugsweise können jedoch mehrere, insbesondere drei Gasanschlüsse vorhanden sein, die umfangmäßig verteilt angeordnet sind. Dies ermöglicht eine gleichmäßige Verteilung des Inertgases innerhalb des umlaufenden Zwischenraums und gewährleistet darüber hinaus, dass die Flutung des Füllschlauchs oberhalb der Abklemmung mit Inertgas rasch erfolgt.

Weitere Vorteile und Merkmale der Erfindung sind den in den Ansprüchen ferner angegebenen Merkmalen sowie dem nachstehenden Ausführungsbeispiel zu entnehmen.

### KURZE BESCHREIBUNG DER ZEICHNUNG

Die Erfindung wird im Folgenden anhand des in der Zeichnung dargestellten Ausführungsbeispiels näher beschrieben und erläutert. Es zeigen:
- Fig. 1: einen Querschnitt durch die erfindungsgemäße Vorrichtung zum Befüllen von Infusionsbeuteln in der Ruhestellung der beweglichen Düse, und
- Fig. 2: einen Querschnitt gemäß Fig. 1, bei dem die bewegliche Düse durch Zentrierbacken nach oben verschoben wurde.

### WEGE ZUM AUSFÜHREN DER ERFINDUNG

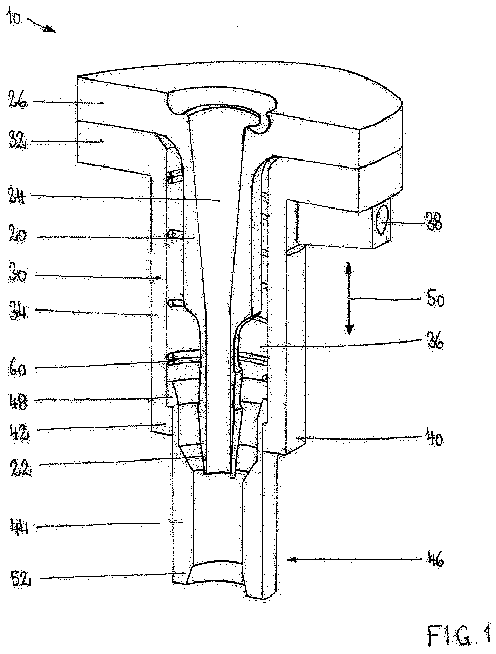

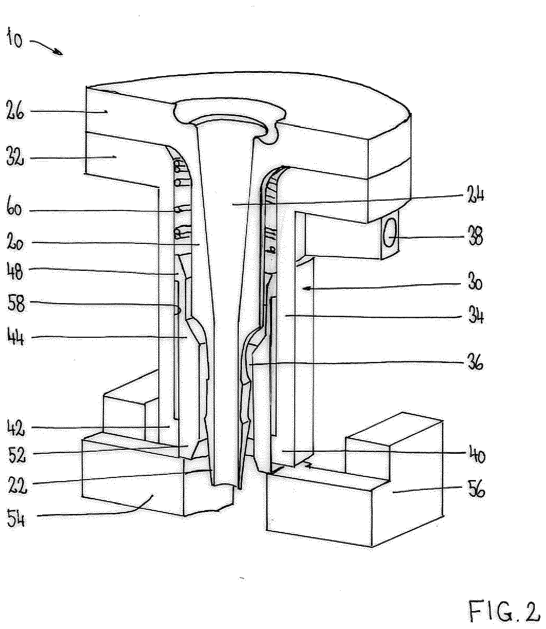

Die erfindungsgemäße Vorrichtung 10 zum Befüllen von Infusionsbeuteln ist in Fig. 1 und 2 dargestellt. Die Vorrichtung 10 besitzt eine Füllnadel 20. Das untere Ende 22 der Füllnadel 20 kann mit einem entsprechenden Ansatz eines Infusionsbeutels - insbesondere mit dem Füllschlauch des Infusionsbeutels - in Kontakt gebracht werden, so dass die zu befüllende Flüssigkeit durch den Innenraum 24 der Füllnadel in den Infusionsbeutel eingefüllt werden kann. Die Füllnadel 20 besitzt an ihrem oberen Ende einen radial auskragenden Anschlussflansch 26. Über den Anschlussflansch 26 kann die Füllnadel 20 an einer hier nicht dargestellten Befüllvorrichtung, insbesondere an einem Füllkopf, befestigt werden. Die Befestigung kann dabei vorzugsweise über eine oder mehrere Schrauben erfolgen.

An dem Anschlussflansch 26 der Füllnadel 20 ist ein Grundhalter 30 mit seinem radial auskragenden Anschlussflansch 32 befestigt. Der Grundhalter 30 besitzt einen Rohrstutzen 34, der außen um die Füllnadel 20 herum verläuft. Zwischen dem Rohrstutzen 34 des Grundhalters 30 und der Füllnadel 20 ist ein umlaufender Zwischenraum 36 vorhanden.

Im vorliegenden Beispielsfall weist die Füllnadel 20 einen etwa kreisförmigen Querschnitt auf, auch der Rohrstutzen 34 des Grundhalters 30 weist einen kreisförmigen Querschnitt auf. Entsprechend ist der umlaufende Zwischenraum 36 im vorliegenden Beispielsfall ringförmig ausgebildet. Grundsätzlich ist die Querschnittsform der Füllnadel 20, insbesondere des unteren Endes 22 der Füllnadel 20 bei der erfindungsgemäßen Vorrichtung frei wählbar. Dadurch kann eine Anpassung des unteren Endes 22 der Füllnadel 20 an unterschiedliche Anschlüsse eines Infusionsbeutels und an unterschiedliche Querschnittsformen der Füllschläuche eines Infusionsbeutels erfolgen. Abhängig von der gewählten Form der Füllnadel 20 kann auch die Form des Rohrstutzens 34 des Grundhalters 30 angepasst werden. Der Rohrstutzen 34 kann also beispielsweise auch einen rechteckigen, eine quadratischen oder einen ovalen Querschnitt aufweisen.

An dem oberen Randbereich des Rohrstutzens 34 ist im vorliegenden Beispielsfall ein Gasanschluss 38 vorhanden. Der Gasanschluss 40 ist unmittelbar unterhalb des Anschlussflansches 32 vorhanden. Durch den Gasanschluss 38 kann der umlaufende Zwischenraum 36 zwischen dem Rohrstutzen 34 und der Füllnadel 20 mit einem Inertgas, insbesondere mit Stickstoff, geflutet werden. Neben dem Gasanschluss 38 können weitere Gasanschlüsse vorhanden sein, die umfangmäßig verteilt angeordnet sein sollten, so dass eine gleichmäßig Flutung des umlaufenden Zwischenraums 36 mit Inertgas möglich ist. Darüber hinaus sollten die einzelnen Gasanschlüsse 38 jeweils am oberen Randbereich des Rohrstutzens 34 des Grundhalters vorhanden sein, damit der umlaufende Zwischenraum 36 möglichst über seine gesamte Längsausrichtung mit Inertgas geflutet werden kann und keine Reste der Umgebungsluft in den oberen Randbereichen des umlaufenden Zwischenraums 36 verbleiben.

Das untere Ende 40 des Rohrstutzens 34 des Grundhalters 30 befindet sich ein Stück oberhalb des unteren Endes 22 der Füllnadel 20. Die Füllnadel 20 steht somit ein Stück weit über den Grundhalter 30 über. Im vorliegenden Beispielsfall weist das untere Ende 40 des Grundhalters 30 eine nach innen kragende umlaufende Schulterausbildung 42 auf. An dieser umlaufenden Schulterausbildung 42 liegt eine bewegliche Düse 44 in ihrer in Fig 1 dargestellten Ruhestellung 46 mit einer umlaufenden Kragnase 48 auf. Die bewegliche Düse 44 verlängert den umlaufenden Zwischenraum 36 nach unten hin. Dabei ragt die bewegliche Düse 44 in ihrer Ruhestellung 46 nach unten über das untere Ende 22 der Füllnadel 20 hinaus.

Die Füllnadel 20 kann zusammen mit dem Grundhalter 30 und der beweglichen Düse 44 in ihrer Längsrichtung 50 verstellt werden und somit höhenverstellbar ausgebildet sein. Auf diese Weise kann die Füllnadel 20 mit dem Grundhalter 30 und der beweglichen Düse 44 im vorliegenden Beispielsfall kontinuierlich in Längsrichtung 50 abgesenkt werden. Während dieses Absenkens der Füllnadel 20 erfolgt gleichzeitig eine Flutung des oberhalb der Abklemmung verbleibenden Füllschlauchs des Infusionsbeutels mit Inertgas. Während des Absenkens kommt es zu einem Kontakt des unteren Endes 52 der beweglichen Düse 44 mit zwei Zentrierbacken 54, 56 (siehe Fig. 2). Die beiden Zentrierbacken 54, 56 dienen somit als Anschlag für die bewegliche Düse 44 und verhindern, dass die bewegliche Düse 44 weiter abgesenkt werden kann. Durch die Zentrierbacken 54, 56 wird die bewegliche Düse 44 aus ihrer Ruhestellung 46 nach oben verschoben, so dass beim weiteren Absenken der Vorrichtung 10 das untere Ende 22 der Füllnadel 20 unterhalb des unteren Endes 52 der beweglichen Düse 44 vorhanden ist. Dabei gleitet die umlaufende Kragnase 48 der beweglichen Düse 44 an der Innenseite 58 des Rohrstutzens 34 entlang. Sobald das untere Ende 22 der Füllnadel 20 so weit abgesenkt wurde, dass ein Kontakt mit dem Füllschlauch des Infusionsbeutels besteht, kann der eigentliche Befüllvorgang gestartet werden.

In dem umlaufenden Zwischenraum 36 ist im vorliegenden Beispielsfall eine Druckfeder 60 vorhanden. Die Druckfeder 60 verläuft entlang der Innenseite 58 des Rohrstutzens und drückt die bewegliche Düse 44 in ihre Ruhestellung 46. Bei einer Auslenkung der beweglichen Düse 44 aus ihrer Ruhestellung 46 heraus, wird die Druckfeder gespannt, so dass nach dem Entfernen der auslenkenden Kraft die Druckfeder die bewegliche Düse wieder in ihre Ruhestellung drückt.

Statt einer kontinuierlichen Absenkung der Vorrichtung wäre es auch möglich, während des Absenkens der Vorrichtung eine oder mehrere Wartepositionen vorzusehen. In diesem Fall könnte die Flutung des Füllschlauchs insbesondere während der Wartepositionen erfolgen.

## Patentansprüche

1. Vorrichtung (10) zum Befüllen eines Infusionsbeutels mit einer Flüssigkeit
- mit einer Füllnadel (20),
- mit einem Grundhalter (30), der so um die Füllnadel (20) herum verläuft, dass zwischen der Füllnadel (20) und dem Grundhalter (30) ein umlaufender Zwischenraum (36) vorhanden ist,
- mit zumindest einem Gasanschluss (38) für Inertgas, durch den Inertgas in den umlaufenden Zwischenraum (36) zwischen Füllnadel (20) und Grundhalter (30) leitbar ist,
- **dadurch gekennzeichnet, dass**
- die Füllnadel (20) an ihrem oberen Ende einen radial auskragenden Anschlussflansch (26) aufweist,
- die Füllnadel (20) mit ihrem Anschlussflansch (26) an einer Befüllvorrichtung anschließbar ist,
- der Grundhalter (30) einen Rohrstutzen (34) aufweist, der außen um die Füllnadel (20) herum verläuft,
- an dem oberen Randbereich des Rohrstutzens (34) ein radial auskragenden Anschlussflansch (32) vorhanden ist,
- der Anschlussflansch (32) des Grundhalters (30) an dem Anschlussflansch (26) der Füllnadel (20) befestigbar ist,
- der Gasanschluss (38) für Inertgas unmittelbar unterhalb des Anschlussflansches (32) des Grundhalters (30) vorhanden ist,
- eine bewegliche Düse (44) vorhanden ist, die längsverschieblich an dem unteren Ende (40) des Grundhalters (30) befestigt ist, wobei die bewegliche Düse (44) in ihrer Ruhestellung (46) über das untere Ende (22) der Füllnadel (20) hinausragt.

2. Vorrichtung nach Anspruch 1,
- **dadurch gekennzeichnet, dass**
- die bewegliche Düse (44) mittels einer Druckfeder (60) längsverschieblich gelagert ist.

3. Vorrichtung nach Anspruch 1 oder 2,
- **dadurch gekennzeichnet, dass**
- die bewegliche Düse (44) in ihrer zurückgeschobenen Position oberhalb des unteren Endes (22) der Füllnadel (20) vorhanden ist.

4. Vorrichtung nach einem der Ansprüche 1 bis 3,
- **dadurch gekennzeichnet, dass**
- die bewegliche Düse (44) durch einen motorischen oder pneumatischen Antrieb aus ihrer Ruhestellung (46) nach oben verschiebbar ist.

5. Vorrichtung nach einem der vorstehenden Ansprüche,
- **dadurch gekennzeichnet, dass**
- die Füllnadel (20) zusammen mit dem Grundhalter (30) und der beweglichen Düse (44) höhenverstellbar ausgebildet ist.

6. Vorrichtung nach einem der vorstehenden Ansprüche,
- **dadurch gekennzeichnet, dass**
- zumindest drei Gasanschlüsse (38) für Inertgas vorhanden sind, durch die Inertgas in den umlaufenden Zwischenraum (36) zwischen Füllnadel (20) und Grundhalter (30) leitbar ist.

## Claims

1. Device (10) for filling an infusion bag with a liquid
- having a filling needle (20),
- having a base holder (30), which extends around the filling needle (20) in such a way that an encircling intermediate space (36) is present between the filling needle (20) and the base holder (30),
- having at least one gas connection (38) for inert gas, through which inert gas is able to be conducted into the encircling intermediate space (36) between the filling needle (20) and the base holder (30), - **characterized in that**
- the filling needle (20) has a radially projecting connection flange (26) at its upper end,
- the filling needle (20) is able to be connected by way of its connection flange (26) to a filling device,
- the base holder (30) has a tubular connecting piece (34) which extends around the outside of the filling needle (20),
- a radially projecting connection flange (32) is present at the upper edge region of the tubular connecting piece (34),
- the connection flange (32) of the base holder (30) is able to be fastened to the connection flange (26) of the filling needle (20),
- the gas connection (38) for inert gas is present directly below the connection flange (32) of the base holder (30),
- a movable nozzle (44) which is fastened in a longitudinally displaceable manner to the lower end (40) of the base holder (30) is present, wherein the movable nozzle (44) projects in its rest position (46) beyond the lower end (22) of the filling needle (20).

2. Device according to Claim 1,
- **characterized in that**
- the movable nozzle (44) is mounted in a longitudinally displaceable manner by means of a compression spring (60).

3. Device according to Claim 1 or 2,
- **characterized in that**
- the movable nozzle (44) is present in its retracted position above the lower end (22) of the filling needle (20).

4. Device according to one of Claims 1 to 3,
- **characterized in that**
- the movable nozzle (44) is displaceable upwards out of its rest position (46) by way of a motorized or pneumatic drive.

5. Device according to one of the preceding claims,
- **characterized in that**
- the filling needle (20) is designed so as to be adjustable in height together with the base holder (30) and the movable nozzle (44).

6. Device according to one of the preceding claims,
- **characterized in that**
- at least three gas connections (38) for inert gas, through which inert gas is able to be conducted into the encircling intermediate space (36) between the filling needle (20) and the base holder (30), are present.

## Revendications

1. Dispositif (10) pour remplir une poche de perfusion avec un liquide
- avec une aiguille de remplissage (20),
- avec un support de base (30) qui s'étend autour de l'aiguille de remplissage (20) de telle sorte qu'un espace intermédiaire périphérique (36) est présent entre l'aiguille de remplissage (20) et le support de base (30),
- avec au moins un raccord de gaz (38) pour un gaz inerte, à travers lequel un gaz inerte peut être conduit dans l'espace intermédiaire périphérique (36) entre l'aiguille de remplissage (20) et le support de base (30), - **caractérisé en ce que**
- l'aiguille de remplissage (20) présente à son extrémité supérieure une bride de raccordement (26) en saillie radiale,
- l'aiguille de remplissage (20) peut être raccordée par sa bride de raccordement (26) à un dispositif de remplissage,
- le support de base (30) présente une tubulure (34) qui s'étend à l'extérieur autour de l'aiguille de remplissage (20),
- une bride de raccordement (32) en saillie radiale est présente sur la zone de bord supérieure de la tubulure (34),
- la bride de raccordement (32) du support de base (30) peut être fixée à la bride de raccordement (26) de l'aiguille de remplissage (20),
- le raccord de gaz (38) pour le gaz inerte est présent directement en dessous de la bride de raccordement (32) du support de base (30),
- une buse mobile (44) est présente, qui est fixée de manière à pouvoir se déplacer longitudinalement sur l'extrémité inférieure (40) du support de base (30), la buse mobile (44) dépassant de l'extrémité inférieure (22) de l'aiguille de remplissage (20) dans sa position de repos (46).

2. Dispositif selon la revendication 1,
- **caractérisé en ce que**
- la buse mobile (44) est montée de manière à pouvoir se déplacer longitudinalement au moyen d'un ressort de compression (60).

3. Dispositif selon la revendication 1 ou 2,
- **caractérisé en ce que**
- la buse mobile (44) est présente dans sa position rétractée au-dessus de l'extrémité inférieure (22) de l'aiguille de remplissage (20).

4. Dispositif selon l'une quelconque des revendications 1 à 3,
- **caractérisé en ce que**
- la buse mobile (44) peut être déplacée vers le haut à partir de sa position de repos (46) par un entraînement motorisé ou pneumatique.

5. Dispositif selon l'une quelconque des revendications précédentes,
- **caractérisé en ce que**
- l'aiguille de remplissage (20) est réalisée pour être réglable en hauteur conjointement avec le support de base (30) et la buse mobile (44).

6. Dispositif selon l'une quelconque des revendications précédentes,
- **caractérisé en ce que**
- au moins trois raccords de gaz (38) pour le gaz inerte sont présents, à travers lesquels le gaz inerte peut être conduit dans l'espace intermédiaire périphérique (36) entre l'aiguille de remplissage (20) et le support de base (30).
